# EUROPÄISCHE PATENTANMELDUNG

(11) **EP 2 327 420 A2**
(43) Veröffentlichungstag der Anmeldung: **01.06.2011**
(21) Anmeldenummer: 10014101.9
(22) Anmeldetag: 21.01.2005
(51) Int. Cl.: A61K 39/275, A61K 35/76, C12N 7/06, C12N 7/08

(54) **Monoparamunitätsinducer basierend auf attenuierten Myxomaviren des Kaninchens**

(30) Priorität: 23.01.2004 DE 102004003572
(62) Teilanmeldung aus: 05701103.3
(71) Anmelder: Bavarian Nordic A/S, 3490 Kvistgaard (DK)
(72) Erfinder: Mayr, Anton, 82319 Starnberg (DE); Mayr, Barbara, 82319 Starnberg (DE)
(74) Vertreter: Pielken, Petra

(57) **Zusammenfassung**

Die vorliegende Erfindung betrifft Monoparamunitätsinducer auf der Basis von paramunisierenden Viren oder Viruskomponenten eines Myxomavirus-Stammes von typisch generalisierend erkrankten Kaninchen, ein Verfahren zur deren Herstellung und deren Verwendung als Arzneimittel zur regulativen Optimierung der paramunisierenden Aktivitäten für die Prophylaxe und Therapie von diversen Dysfunktionen bei Mensch und Tier.

## Beschreibung

Die Erfindung betrifft Monoparamunitätsinducer auf der Basis von paramunisierenden Viren oder Viruskomponenten, **dadurch gekennzeichnet, dass** die Viren oder Viruskomponenten aus einem attenuierten Myxomavirus-Stamm des Kaninchens stammen, ein Verfahren zur Herstellung der Monoparamunitätsinducer und deren Verwendung als Arzneimittel.

Das körpereigene Immunsystem von hochentwickelten Organismen, insbesondere das der Säuger und Vögel, umfasst einen antigenspezifischen sowie einen antigenunspezifischen Teil. Beide Teile des Immunsystems sind miteinander vernetzt und stehen dabei in gegenseitiger Wechselwirkung. Die antigenspezifischen Mechanismen sind für den Aufbau der Immunität, die antigenunspezifischen für den Aufbau der Paramunität verantwortlich. Paramunität bezeichnet den Zustand eines gut regulierten und optimal funktionierenden unspezifischen Abwehrsystems, verbunden mit einem schnell entstandenen, zeitlich limitierten, erhöhten Schutz gegenüber einer Vielzahl unterschiedlicher Erreger, Antigene und anderer Noxen. Grundlage für die Entstehung von Paramunität sind aus historischen und funktionellen Gründen die, aus phylogenetischer Sicht alten, sogenannten primitiven, nichtselektiven und bedingt selektiven paraspezifischen Abwehrmechanismen.

Die paraspezifischen Aktivitäten des antigenunspezifischen Immunsystems (engl.: "innate immune system") umfassen nichtselektive Schutzelemente, wie zum Beispiel fremdmaterialfressende Organellen, sowie bedingt selektive Schutzelemente, wie zum Beispiel Mikro- und Makrophagen, natürliche Killerzellen, dendritische Zellen und lösliche Faktoren wie Zytokine, die entsprechend ihrer Genese erregerunspezifisch bzw. antigenunspezifisch reagieren.

Paraspezifische Aktivitäten sind im betreffenden Organismus unmittelbar nach Antigenkontakt zu verzeichnen, während die Wirkungen des antigenspezifischen Immunsystems erst nach Tagen oder Wochen eintreten.

Bei den höher organisierten Lebewesen wird hierdurch zusätzlich Zeit gewonnen, um spezifische Abwehrsysteme gegenüber den Noxen aufzubauen, die noch nicht eliminiert werden konnten und antigene Eigenschaften besitzen.

Der Nutzen der Paramunisierung, d.h. der paraspezifischen Aktivitäten des Immunsystems für die Prophylaxe und Therapie bei einem Patienten hat sich seit deren Entwicklung in immer deutlicher Weise gezeigt (Anton Mayr, "Paramunisierung: Empirie oder Wissenschaft", Biol. Med., Aufl. 26(6): 256-261, 1997). Die paraspezifische Abwehr ermöglicht es dem Organismus, sich bei Konfrontation mit den unterschiedlichsten Fremdstoffen, Infektionserregern, Toxinen und transformierten körpereigenen Zellen sofort zur Wehr zu setzen.

Zwischen den paraspezifischen und den spezifischen Aktivitäten des Immunsystems bestehen enge Wechselwirkungen, wobei der Informationsfluss in der Regel vom zuerst reagierenden, paraspezifischen zum später einsetzenden, spezifischen Teil des Immunsystems verläuft (z.B. bei der Antigenvermittlung). Die paraspezifische Abwehr des Organismus kann auf diese Weise bei Infektionen mit besonders virulenten Pathogenen die Zeit bis zur Ausbildung der spezifischen Immunität (z.B. Antikörper, Immunzellen) überbrücken.

Die paraspezifische Immunabwehr ist ein physiologischer Vorgang und lässt sich als "primäre Kontrolle" bei der Auseinandersetzung mit der Umwelt definieren. Sie ist nicht nur für die niederen Organismen, sondern insbesondere auch für die höher- und hochentwickelten Wirbeltiere unersetzlich. Primäre kongenitale Defekte in diesem biologischen Abwehrsystem führen zu lebensbedrohenden Situationen. Als Beispiel ist das "Chediak-Steinbrinck-Higashi-Syndrom" des Menschen zu nennen, das durch Granulozytendefekte und Dysfunktionen der natürlichen Killerzellen (NK-Zellen) charakterisiert ist und in den meisten Fällen bis zur Vollendung des 10. Lebensjahrs zum Tode des Patienten führt.

Der Zustand der Paramunität ist durch eine erhöhte Phagozytoserate, eine erhöhte Funktion der spontanen zellvermittelten Zytotoxizität (NK-Zellen) und eine erhöhte Aktivität sonstiger lymphoretikulärer Zellen gekennzeichnet. Gleichzeitig kommt es zur Freisetzung bestimmter Zytokine, die sowohl mit den zellulären Elementen als auch untereinander stimulierend und/oder hemmend (z.B. über Repressormechanismen) wirken. Dieses eng vernetzte und stufenweise reagierende biologische System der Paramunität mit seinen unterschiedlichen Akzeptor-, Effektor- und Zielzellen sowie den signalübertragenden Zytokinen ist daneben intensiv mit dem Hormon- und Nervensystem verbunden. Es stellt dadurch einen wichtigen Bestandteil des Kommunikations-, Interaktions- und Regulationsnetzwerkes dar.

Die Einleitung der Paramunität erfolgt durch Paramunisierung. Darunter versteht man die medikamentöse Aktivierung der zellulären Elemente des paraspezifischen Teils des Immunsystems und der damit verbundenen Bildung von Zytokinen, mit dem Ziel, Dysfunktionen zu beseitigen, den nichterreger- und nicht antigenspezifischen Schutz eines Individuums schnell zu erhöhen (optimale Bioregulation), eine durch Stressfolgen oder anderweitig (z.B. medikamentös) entstandene Immunsuppression oder Immunschwäche zu beseitigen, Defizite zu reparieren und/oder regulatorisch zwischen Immun-, Hormon- und Nervensystem zu wirken. Dies bedeutet, dass je nach Art der Paramunisierung und der Reaktionslage, wie zum Beispiel der Gesundheitslage des Patienten, bestimmte unspezifische, körpereigene Abwehrvorgänge gesteigert, ergänzt oder auch gedämpft werden können.

Für die Paramunisierung verwendet man Paramunitätsinducer, an die bestimmte Unschädlichkeits- und Wirksamkeitskriterien gestellt werden, wodurch sie sich von Immunstimulanzien unterscheiden. Der Paramunitätsinducer *per se* ist weder mit einem Abwehrstoff, noch mit einer Chemikalie, einem Antibiotikum, Vitamin oder Hormon vergleichbar. Vielmehr aktiviert er über einen stufenweisen Mechanismus das paraspezifische Immunsystem, so dass dieses ausreichend zelluläre und humorale Abwehrmechanismen mobilisiert. Der Paramunitätsinducer wirkt hierbei sowohl regulierend als auch reparierend hinsichtlich der Immunabwehr. Bezüglich der Wirkungsweise von Paramunitätsinducer ist bekannt, dass sie von phagozytierenden Zellen (Akzeptorzellen) aufgenommen werden, die dadurch aktiviert werden und Mediatoren entlassen, welche wiederum Effektorzellen mobilisieren. Diese schalten schließlich die Regulationsmechanismen der paraspezifischen Abwehr ein.

Multiple Paramunitätsinducer auf der Basis von Kombinationen zweier oder mehrerer Pockenviruskomponenten, die sich aus unterschiedlichen Pockenvirus-Stämmen mit paramunisierenden Eigenschaften ableiten, sind in der europäischen Patentschrift EP 0 669 133 B1 beschrieben.

Die vorliegende Erfindung basiert auf den paramunisierenden Eigenschaften von attenuierten Myxomaviren und/oder ihren Virusbestandteilen.

Eine Attenuierung bezeichnet die Umwandlung der Eigenschaften eines Infektionserregers, die zur Abschwächung des Erregers führen (engl. "attenuate" = abschwächen, mildern). Veränderungen von Infektionserregern treten in der Natur spontan häufig auf, wobei die Zeitspannen über viele Jahrhunderte reichen können.

Experimentell kann man die Wandlungsfähigkeit von Infektionserregern, sich an Umweltveränderungen anzupassen, nutzen und die zur Attenuierung benötigte Zeitspanne, z.B. durch Dauerpassagen in bestimmten Wirtssystemen, drastisch verkürzen. Bisher nutzte man die Attenuierung zur Gewinnung von avirulenten Impfstämmen sowie von unschädlichen Paramunitätsinducern.

In der Regel führt eine Attenuierung zum Verlust der Virulenz und Kontagiosität, der Verminderung der immunisierenden Eigenschaften und des Wirtsspektrums sowie zu geringen Veränderungen im Erregergenom mit Auftreten von Deletionen, bevorzugt in den terminalen Bereichen. Parallel kommt es in der Regel zu einer Zunahme der paramunisierenden Aktivitäten des modifizierten Erregers.

In seltenen Fällen kann eine Attenuierung, vor allem beim Versuch einer experimentellen Attenuierung durch gentechnische Manipulationen, auch zu einer Erhöhung der Virulenz und Kontagiosität führen.

Myxomaviren sind die Erreger der Myxomatose, eine zyklisch verlaufende, kontagiöse Virusallgemeinkrankheit der Wild- und Hauskaninchen, die durch generalisierte, teilweise hämorrhagische Unterhautödeme am Kopf und über den ganzen Körper, mit Bevorzugung der Analgegend, der Vulva und des Schlauches, wie keine andere Infektionskrankheit charakterisiert ist. Wird die Myxomatose in ein bisher seuchenfreies Land neu eingeschleppt, verläuft sie rasch und tödlich. Nach Sesshaftwerden des Virus verändert sich der Seuchencharakter bis hin zu klinisch inapparenten Infektionen (Mayr A.: Medizinische Mikrobiologie, Infektions- und Seuchenlehre, 7. Aufl., Enke-Verlag, Stuttgart, 2002).

Die Krankheit ist unter amerikanischen Baumwollschwanzkaninchen der Gattung *Sylvilagus,* die ausschließlich die neue Welt besiedeln, weit verbreitet. Diese Wildkaninchen bilden das einzige natürliche Reservoir der Seuche. Die Infektion verläuft bei ihnen in einer milden Form. Dagegen besitzt die Erkrankung bei europäischen Wild- und Hauskaninchen der Gattung *Oryctolagus,* die auch in Australien heimisch sind, eine fast 100%ige Sterblichkeit bei Neueinschleppung des Erregers.

Das natürliche Wirtsspektrum des Myxomavirus (Genus *Leporipoxvirus*) ist eng begrenzt. Im Allgemeinen vermehrt sich das Virus nur in amerikanischen Baumwollschwanzkaninchen und in europäischen Haus- und Wildkaninchen. Vereinzelt wurden allerdings auch Infektionen in europäischen Wildhasen beobachtet. Übertragungsversuche auf andere Tierarten und auf den Menschen verliefen negativ.

Der vorliegenden Erfindung liegt die Aufgabe zugrunde, neue Monoparamunitätsinducer für die Humanmedizin und Veterinärmedizin bereitzustellen. Eine weitere Aufgabe der vorliegenden Erfindung besteht darin, ein Verfahren zur Herstellung solcher Monoparamunitätsinducer bereitzustellen. Ferner ist es Aufgabe der vorliegenden Erfindung, pharmazeutische Zusammensetzungen zur Verwendung als Arzneimittel auf der Basis von Monoparamunitätsinducern bereitzustellen.

Entsprechend betrifft die vorliegende Erfindung Monoparamunitätsinducer auf der Basis von paramunisierenden Viren oder Viruskomponenten, **dadurch gekennzeichnet, dass** die Viren oder Viruskomponenten von einem attenuierten Myxomavirus-Stamm des Kaninchens stammen. Vorzugsweise umfassen die Viruskomponenten paramunisierende Virushüllen oder aberrante Formen von Virushüllen eines attenuierten Myxomavirus-Stammes. Bevorzugte Stämme, welche die erfindungsgemäßen paramunisierenden Eigenschaften aufweisen, sind die Stämme M-2, M-7, Lausanne, Aust/UriarraNerg-86/1. Die Stämme M-7, Lausanne, Aust/Uriarra/Verg-86/1 sind auch für die Herstellung von Lebend-Vaccinen geeignet, da sie nur partiell in ihrer Virulenz abgeschwächt sind, um ausreichend immunisierend zu wirken.

Besonders bevorzugt ist ein Monoparamunitätsinducer der auf dem Myxomavirus-Stamm M-2 basiert. Ein attenuierter Myxomavirus-Stamm, hergestellt nach dem unten beschriebenen erfindungsgemäßen Verfahren ist bei der Hinterlegungsstelle der Public Health Laboratory Service (PHLS), Centre for Applied Microbiology & Research, European Collection of Animal Cell Cultures (ECACC), Salisbury, Wiltshire, United Kingdom mit der Hinterlegungsnummer 03121801 hinterlegt worden.

Die Erfindung betrifft ferner ein Verfahren zur Herstellung von Monoparamunitätsinducern basierend auf einem attenuierten Myxomavirus-Stamm des Kaninchens. Dafür werden zunächst Myxomaviren aus infiziertem Gewebe eines typisch an einer generalisierten Myxomatose erkrankten Kaninchens isoliert. Anschließend erfolgt eine Adaptierung des Virus an ein permissives Zellsystem, d.h. an ein Zellmaterial, das die Vermehrung des Virus erlaubt, wie beispielsweise Zellkulturen, bebrütete Hühnereier oder auch Versuchstiere. Insbesondere können Zellen des natürlichen Wirts oder einer mit dem Wirt nahe verwandten Spezies für die Adaptierung verwendet werden. Als permissive Zellsysteme für Myxomaviren eignen sich beispielsweise Hühnerembryofibroblasten (FHE) wie auch aus Kaninchennieren oder -hoden hergestellte Zellkulturen.

Als erfindungsgemäß bevorzugt ist die Adaptierung des isolierten Myxomavirus auf die Chorioallantoismembran (CAM) bebrüteter Hühnereier über eine oder mehrere Passagen, bevorzugt über 2 bis 6 Passagen und besonders bevorzugt über 3 Passagen. Dafür werden zur Adaptierung die isolierten Viren auf die CAM geimpft und über Passagierung auf der CAM vermehrt.

Vorzugsweise werden die Myxomaviren aus infiziertem Gewebe durch Vermehrung in einem permissiven Zellsystem zunächst isoliert. Dafür kann ein permissives Zellsystem beispielsweise mit infiziertem, durch Aufschluß gewonnenem Gewebehomogenat beimpft werden. Anschließend werden die durch erste Vermehrung gewonnenen Viren entweder weiter an denselben, permissiven Zellsystemtypen, der auch bereits für die Isolierung verwendet wurde, adaptiert, oder es wird ein anderes, weiteres permissives Zellsystem verwendet. Bevorzugt ist die Adaptierung des Virus an denselben permissiven Zellsystemtypen, der auch für die Isolierung verwendet wird. Somit werden vorzugsweise Myxomaviren aus infiziertem Gewebe durch Vermehrung in einem permissiven Zellsystem isoliert und anschließend an das permissive Zellsystem durch weitere Passagen adaptiert. Besonders bevorzugt ist eine erste Anzucht bzw. Isolierung der Myxomaviren durch Vermehrung auf der CAM bebrüteter Hühnereier und nachfolgender Adaptierung des Virus auf der CAM über weitere Passagen, vorzugsweise über weitere 2 Passagen. Alternativ kann für die Anzucht und/oder Adaptierung aber auch die Allantoisflüssigkeit bebrüteter Hühnereier verwendet werden.

Die eigentliche Attenuierung erfolgt dann durch Dauerpassagen auf einer oder mehreren permissiven Zellkulturen, bis eine Attenuierung bzw. der gewünschte Attenunierunggrad des Virus erreicht ist. Dafür können zur Vermehrung des Virus zunächst verschiedene permissive Zellsysteme ausprobiert werden und anschließend ein oder mehrere Zellsysteme, in denen die höchsten Infektiositätstiter erzielt werden, für weitere Passagierungen ausgewählt werden. Für eine Attenuierung durch Dauerpassagen eignen sich sowohl primäre als auch sekundäre Zellkulturen wie auch permanente bzw. kontinuierliche Zelllinien. So kann eine Attenuierung durch Vermehrung in primären oder sekundären Hühnerembryofibroblasten-(FHE-) Kulturen oder in Kulturen permanenter FHE-Zellen erfolgen.

Erfindungsgemäß bevorzugt wird für die Attenuierung des Myxomavirus das Virus in einer permanenten Zellkultur, insbesondere einer Vero-Zellkultur, passagiert bzw. vermehrt, bevorzugt über 80 bis 150 Passagen und besonders bevorzugt über 120 Passagen. Alternativ oder zusätzlich wird das Virus erfindungsgemäß auf einer binären, permanenten Zelllinie passagiert, wobei bevorzugt AVIVER-Zellen verwendet werden. Diese Zellen bzw. Zellkultur wurde durch eine Zellfusion zwischen Hühnerembryfibroblasten (FHE) und Vero-Affennierenzellen erhalten. Für die erfindungsgemäße Attenuierung des Myxomavirus werden vorzugsweise die isolierten und adaptierten Viren in einem ersten Schritt in Vero-Zellkulturen passagiert, die Viren anschließend in eine binäre AVIVER-Zellkultur überführt und in diesen vorzugsweise über 10 bis 50 Passagen, insbesondere über 20 bis 30 Passagen, und besonders bevorzugt über 25 Passagen vermehrt.

Anschließend kann das attenuierte Myxomavirus noch zusätzlich über weitere Attenuierungspassagen vermehrt werden. Eine Weitervermehrung der Viren erfolgt vorzugsweise über weitere Passagierung in Vero-Affennierenzellen, insbesondere über 100 bis 200 Passagen.

Ein besonders bevorzugter, weiterer Verfahrensschritt ist eine zusätzliche Inaktivierung des attenuierten Myxomavirus. Eine Inaktivierung kann durch chemische Behandlung, Bestrahlung, Hitze- oder pH-Einwirkung erfolgen, insbesondere durch eine chemische Behandlung mit Beta-Propiolacton. Durch die Behandlung der attenuierten Myxomaviren mit Beta-Propiolacton werden die paraspezifischen Aktivitäten noch gesteigert, während die nach der Attenuierung ggf. noch vorhandenen immunisierenden Eigenschaften verloren gehen.

Eine besonders bevorzugte Ausführungsform des erfindungsgemäßen Verfahrens umfasst die folgenden Schritte:
- Isolieren von Myxomaviren aus infiziertem Gewebe eines typisch an einer generalisierten Myxomatose erkrankten Kaninchens durch Vermehrung auf der Chorioallantoismembran (CAM) bebrüteter Hühnereier und nachfolgender Adaptierung des Virus an die CAM über weitere 2 Passagen;
- Attenuieren der isolierten Viren durch Passagierung in Vero-Zellkulturen, vorzugsweise über 120 Passagen;
- Überführung der Viren in eine binäre AVIVER-Zellkultur, wobei die AVIVER-Zellen durch eine Zellfusion zwischen Hühnerembryofibroblasten (FHE)- und Vero-Affennierenzellen erhalten wurden, und Attenuierung des Virus in dieser Zellkultur über 10 bis 50, vorzugsweise 25, Passagen,
- nachfolgende Rückübertragung des Virus auf Vero-Affennierenzellen und Vermehren der Viren durch weitere Attenuierungspassagen in den Vero-Zellen, vorzugsweise über etwa 150 Passagen;
   und wahlweise
- Inaktivieren der attenuierten Myxomaviren durch Behandlung mit Beta-Propiolacton, wobei durch diese Behandlung die paraspezifischen Aktivitäten gesteigert werden, während die nach der Attenuierung noch vorhandenen immunisierenden Eigenschaften verloren gehen.

Die Attenuierung durch Dauerpassage wird in der Regel durch 3 bis 5 Plaque-Endverdünnungen abgeschlossen. Nach Gewinnung und Austestung verschiedener Klone werden diejenigen Klone zur Weitervermehrung ausgewählt, mit denen die höchsten Infektiositätstiter erzielt und mit denen eine hohe paramunisierende Wirksamkeit - z.B. im VSV challenge Test in der Babymaus - nachgewiesen werden. Diese Vorgehensweise soll insbesondere für die Bereitstellung von genetisch einheitlichem Virusmaterial für die weitere Verwendung sorgen.

Der Ausdruck "*Attenuierung*", wie er im Zusammenhang mit dieser Erfindung verwendet wird, bezeichnet die experimentelle Veränderung des ursprünglich virulenten Myxomavirus in die modifizierte Form, bei gleichzeitiger Steigerung der paramunisierenden Eigenschaften. Nachweisbar ist die Attenuierung durch eine oder mehrere der folgenden Eigenschaften:
- Verminderung bzw. Abschwächung oder Verlust der Virulenz für europäische Haus- und Wildkaninchen (Gattung *Oryctolagus caniculus csp.*),
- Abschwächung oder Verlust der Kontagiosität,
- Einengung des Wirtsspektrums in Zellkulturen,
- Änderung der immunisierenden Eigenschaften
- Erwerb paramunisierender, kurzzeitig protektiver Aktivitäten.

Eine Attenuierung kann zu Deletionen im terminalen Bereich des Myxomavirusgenoms führen. Häufig wird mit zunehmendem Attenuierungsgrad eine zunehmende Anzahl von Deletionen im viralen Genom beobachtet.

Der Attenuierungsgrad kann im Laufe der Passagierungen durch entsprechende, geeignete Wirksamkeitstests, wie sie im Stand der Technik bekannt sind (vgl. z.B. US 6,805,870, Spalte 12, sowie die weiteren, dort zitierten Literaturhinweise) und durch Klonierung überprüft und kontrolliert werden.

Die vorliegende Erfindung betrifft ferner attenuierte Myxomaviren, die durch das erfindungsgemäße Verfahren erhältlich sind, pharmazeutische Zusammensetzungen, die das attenuierte Myxomavirus bzw. den erfindungsgemäßen Myxomavirus-Monoparamunitätsinducer umfassen, sowie die Verwendung der Myxomavirus-Monoparamunitätsinducer zur Aktivierung des paraspezifischen Immunsystems in einem Säugetier bzw. die Verwendung des attenuierten Virus zur Herstellung eines entsprechenden Arzneimittels.

Aufgrund der überraschenden paramunisierenden Eigenschaften eignen sich die erfindungsgemäßen Myxomavirus-Monoparamunitätsinducer zur Behandlung und/oder zur Prophylaxe von Immunsystem-Dysfunktionen, Immunsuppression, Immunschwäche-Erkrankungen, Dysfunktionen der Homöostase zwischen Hormon-, Kreislauf-, Stoffwechsel- und Nervensystem, neonataler Infektionsbedrohung, Tumorerkrankungen, Viruserkrankungen, bakteriellen Erkrankungen, therapieresistenten infektiösen Faktorenkrankheiten, viralen und bakteriellen Mischinfektionen, chronischen Manifestationen infektiöser Prozesse, Leberkrankheiten unterschiedlicher Genese, chronischen Hautkrankheiten, Herpeskrankheiten, chronischen Hepatitiden, grippalen Infekten, Endotoxinschäden.

Die erfindungsgemäßen Monoparamunitätsinducer sind generell unschädlich für die Umwelt und wirksam im Sinne einer Paramunisierung für Säugetiere, wie z.B. Mensch, Pferd, Hund, Katze, Schwein, für Vögel sowie auch für Reptilien, wie z.B. Echsen, Schlangen, Schildkröten. Sie sind daher für die Anwendung in der Human- und Veterinärmedizin besonders gut geeignet.

Darüberhinaus weisen die erfindungsgemäßen Monoparamunitätsinducer eine sehr gute paramunisierende Wirksamkeit mit hoher Potenz auf. Sie sind mit dem erfindungsgemäßen Verfahren in geeigneter Weise herstellbar und sicher für die Anwendung im medizinischen Bereich. Durch die Attenuierung der Myxomaviren nehmen die immunisierenden Eigenschaften der Myxomaviren ab, während die paraspezifischen Aktivitäten zunehmen. Die erfindungsgemäßen Monoparamunitätsinducer haben daher keine immunisierenden sondern paramunisierenden Eigenschaften, was eine mehrmalige und kontinuierliche Anwendung ermöglicht. Diese paramunisierenden Eigenschaften des Myxomavirus des Kaninchens bzw. seiner paramunisierenden Viruskomponenten sind überraschend und waren nicht vorhersehbar.

Der Ausdruck "*Paramunisierung*" wie er im Zusammenhang mit dieser Erfindung verwendet wird, bezeichnet die medikamentöse Aktivierung der zellulären Elemente des paraspezifischen Immunsystems und die damit verbundene Bildung bzw. Freisetzung von Zytokinen mit dem Ziel, Dysfunktionen zu beheben, den nichterreger- und nichtantigenspezifischen Schutz eines Individuums schnell zu erhöhen und regulatorisch zwischen Immun-, Hormon-, Nerven- und Gefäßsystem zu wirken. Eine Paramunisierung führt zum Schutzzustand der Paramunität.

Der Ausdruck "*Paramunität*" wie er im Zusammenhang mit dieser Erfindung verwendet wird, bezeichnet den aktiv erworbenen Zustand eines optimal regulierten und funktionierenden paraspezifischen Abwehrsystems, verbunden mit einem schnell entstandenen, zeitlich limitierten Schutz gegenüber einer Vielzahl von Erregern, Antigenen und anderen Noxen. Die Phagozytoserate, die Funktion der NK-Zellen (natürliche Killerzellen) und die Aktivität sonstiger lymphoretikulärer Zellen (z.B. dendritischer Zellen) sind bis zum physiologischen Optimum erhöht.

Der Ausdruck "*Paramunitätsinducer*" wie er im Zusammenhang mit dieser Erfindung verwendet wird, bezeichnet ein pyrogenfreies, nicht toxisches Arzneimittel, das dazu bestimmt ist, bei Mensch und Tier zur Erzeugung und Regulierung körpereigener Abwehr- und Schutzmechanismen im Sinne einer Paramunisierung angewendet zu werden.

Der Ausdruck "*Myxomavirus-Monoparamunitätsinducer*" wie er im Zusammenhang mit dieser Erfindung verwendet wird, bezeichnet ein Arzneimittel das auf attenuierte Myxomaviren des Kaninchens bzw. einem attenuierten Myxomavirus-Stamm basiert, einschließlich der paramunisierenden Viruskompontenten und der Bestandteile davon, die in einem Organismus, bevorzugt in einem Säugetier (z.B. Mensch), den Zustand der Paramunität erzeugen.

Der Ausdruck "*Myxomavirus*" wie er im Zusammenhang mit dieser Erfindung verwendet wird, bezeichnet die Spezies des Myxomatosevirus des Genus *Leporipoxvirus.* Das Myxomavirus gehört zur Unterfamilie der *Chordopoxviridae* und zur Familie der *Poxviridae* (Pockenviren).

Unter dem Begriff *"paramunisierende Viruskomponenten"* wie er im Zusammenhang mit dieser Erfindung verwendet wird, werden eine Vielzahl von viralen Strukturen, die von einem Myxomavirus mit paramunisierenden Eigenschaften abgeleitet sind umfasst, beispielsweise vermehrungsfähige oder inaktivierte frisch isolierte Myxomaviren, vermehrungsfähige oder inaktivierte rekombinante Myxomaviren, die sich von frisch isolierten Myxomaviren ableiten, Virushüllen, die abgetrennten Hüllen sowie Spaltprodukte und aberrante Formen dieser Hüllen, einzelne native oder rekombinante Polypeptide oder Proteine, insbesondere Membran- und Oberflächenrezeptoren, die in frisch isolierten Myxomaviren vorkommen oder von einem genetisch modifizierten Myxomavirus oder einem Teil seiner genetischen Information rekombinant exprimiert werden.

In den Tabellen 1 bis 4 sind die klinischen Ergebnisse mit dem Myxomavirus-Monoparamunitätsinducer PIND-MYXO basierend auf dem attenuierten Myxomatose-Zellkulturvirus, Stamm M-2, beim Menschen zusammengestellt.
Tabelle 1 zeigt die klinischen Ergebnisse bei der prophylaktischen Anwendung des Myxomavirus-Monoparamunitätsinducer PIND-MYXO beim Menschen.
Tabelle 2 zeigt die klinischen Ergebnisse bei der therapeutischen Anwendung des Myxomavirus-Monoparamunitätsinducer PIND-MYXO beim Menschen.
Tabelle 3 zeigt die Wirkung einer Paramunisierung mit Myxomavirus-Monoparamunitätsinducer (PIND-MYXO) bei Patienten mit niedrigen Immunparametern (7 Tage nach PIND-MYXO-Applikation)
Tabelle 4 zeigt die Wirkung einer Paramunisierung mit Myxomavirus-Monoparamunitätsinducer (PIND-MYXO) bei Patienten mit erhöhten Immunparametern (7 Tage nach PIND-MYXO-Applikation)

Die Erfindung basiert auf den überraschenden Befund, dass attenuierte Myxomaviren des Kaninchens oder deren paramunisierenden Bestandteile in der Lage sind, sehr gute paramunisierende Eigenschaften in einem Empfängerorganismus hervorzurufen, die zum Schutzzustand der Paramunität führen. Grundlage für diese Erfindung war die erstmals gelungene Attenuierung von Myxomaviren des Kaninchens in Zellkulturen.

Vorzugsweise basieren die erfindungsgemäßen Monoparamunitätsinducer auf lyophilisierten, attenuierten und inaktivierten Myxomaviren des Kaninchens oder deren paramunisierenden Viruskomponenten. Vorzugsweise stammen die erfindungsgemäßen attenuierten Myxomaviren oder deren Viruskomponenten von einem Myxomavirus-Stamm oder mehreren unterschiedlichen attenuierten Myxomavirus-Stämmen. Es ist dabei bevorzugt, dass die erfindungsgemäßen Monoparamunitätsinducer Kombinationen von einem oder mehreren Myxomavirusstämmen oder deren paramunisierenden Viruskomponenten umfassen.

Die paramunisierenden Eigenschaften, welche durch die Applikation von Myxomavirus-Monoparamunitätsinducer in einem Säugetier, wie zum Beispiel beim Menschen, hervorgerufen werden, sind besonders nützlich zur Beseitigung von Dysfunktionen, zur Erhöhung des nichtantigenspezifischen Schutzes eines Individuums, zur Beseitigung einer durch Stressfolgen oder anderweitig (z.B. medikamentös) entstandenen Immunsuppression oder Immunschwäche und um regulatorisch zwischen dem Immun-, Hormon- und Gefäßsystem zu wirken.

Die Erfindung basiert ferner auf einer gelungenen Attenuierung eines Myxomavirus-Stammes durch Passagierung über Zellkulturen, wobei die virulenten und/oder immunisierenden Eigenschaften des Myxomavirus-Stammes vermindert werden bzw. verloren gehen. Eine zusätzliche Inaktivierung der Myxomaviren kann desweiteren durch Bestrahlung, Hitze- oder pH-Einwirkung erfolgen oder, besonders bevorzugt, durch eine chemische Behandlung mit Beta-Propiolacton. Die Monoparamunitätsinducer basieren auf attenuierten, lyophilisierten Myxomaviren, wobei auch einzelne Viruskomponenten eines Myxomavirus, die geeignet sind, paramunisierende Aktivitäten in einem Organismus hervorzurufen, von der Erfindung umfasst sind.

Im Folgenden soll eine Ausführungsform des erfindungsgemäßen Herstellungsverfahrens der Monoparamunitätsinducer auf der Basis eines isolierten und attenuierten Myxomavirus-Stammes des Kaninchens über Zellkulturpassagierung dargestellt werden. Das Herstellungsverfahren ist hierbei nicht auf diesen bevorzugten Stamm beschränkt, sondern gleichermaßen auf andere Myxomavirus-Stämme des Kaninchens übertragbar. Auch umfasst von der vorliegende Erfindung sind rekombinante Formen eines Myxomavirus-Stammes, die mittels genetischer Modifikation hergestellt worden sind. Bevorzugt sind hierbei rekombinante Myxomavirus-Stämme, bei denen ein oder mehrere Abschnitte im Genom, welche für Zytokin-Rezeptoren kodieren, durch eine Modifikation in Form einer Addition, Substitution oder Deletion modifiziert wurden, wobei durch die Modifikation die Rezeptoreigenschaften des Zytokin-Rezeptors verloren geht. Vorzugsweise sind dies die Genabschnitte, die für die Rezeptoren für Interferone (IFN), Interleukine (IL) und Tumornekrosefaktoren (TFN) kodieren, insbesondere für IFN-α-R, IFNγ-R, TNF-R, IL-1-R, IL-2-R, IL-6-R und IL-12-R.

Weiterhin sollen die hier angegebenen Zahlenwerte bezüglich der Inkubationsdauer oder der Passagierungszahl über Zellkulturen nicht als beschränkend aufgefasst werden. Geringfügige Modifikationen dieser Parameter und Modifikationen, die für den Fachmann ersichtlich sind und auch zur einer Präparation von attenuierten Myxomaviren führen, sind von dieser Erfindung gleichermaßen umfasst.

Eine bevorzugte Ausführungsform der vorliegenden Erfindung betrifft die gelungene Attenuierung des Myxomavirus-Stammes M-2. Der Myxomavirus-Stamm M-2 wurde aus europäischen Wildkaninchen, die an Myxomatose erkrankt waren, isoliert (Herrlich A., Mayr A. und Munz E.: "Die Pocken", 2. Aufl., Georg Thieme Verlag, Stuttgart, 1967). Die veränderten Hautzellen, die aus dem Unterhautgewebe des erkrankten Kaninchens gewonnen worden sind, werden nach Aufschluss auf die Chorioallantoismembran (CAM) von vorzugsweise 10-12 Tage lang bebrüteter Hühnereier geimpft. Die Myxomaviren werden über 2 bis 6 Passagen, vorzugsweise über drei Passagen, auf der Chorioallantoismembran weiter vermehrt bzw. adaptiert (CAM-Passagen; Verfahren siehe Herrlich A., Mayr A. und Munz E.: "Die Pocken", 2. Aufl., Georg Thieme Verlag, Stuttgart, 1967). Die 2. bis 6. Passage, vorzugsweise die dritte CAM-Passage, dient als Ausgangsmaterial für die weitere Attenuierung des Myxomavirus in Zellkulturen. Die Attenuierung erfolgt nach Adaptierung der Viren in der Chorioallantoismembran (erkennbar durch typische Herde auf der Chorioallantoismembran) in 3 Stufen. In Stufe 1 werden 80 bis 150, vorzugsweise 120 kontinuierliche sogenannte Endverdünnungs-Passagen in Vero-Zellen durchlaufen (Vero-Zellen, ATCC CCL-81). Nach Durchlaufen dieser Passagen ist das Myxomavirus in seiner Virulenz abgeschwächt.

In einer 2. Stufe wird nach der 80. bis 150. Passage, vorzugsweise nach der 120. Passage in Vero-Zellen, die Virussuspension auf sogenannte AVIVER-Zellen übertragen und über 10 bis 50 Passagen, vorzugsweise über 20 bis 30, insbesondere über 25 Passagen, weitergeführt. AVIVER-Zellen werden durch Zellfusion zwischen Hühnerembryofibroblasten (FHE)- und Vero-Zellen gewonnen und werden als binäre permanente Zellkultur bezeichnet.

Die letzte Passage über AVIVER-Zellen, vorzugsweise die 25. Passage, wird auf Vero-Zellen zurückübertragen und wird in der 3. Stufe der Attenuierung für weitere 100 bis 200 Passagen, vorzugsweise etwa 157 Passagen, in Vero-Zellen weitergeführt. Auf diese Weise wird das Myxomavirus über insgesamt mehr als 300 Zellkulturpassagen vermehrt. Nach der 3. Stufe der Vermehrung des Myxomavirus in Zellkulturen ist das Myxomavirus ausreichend attenuiert.

Für die Anzucht der VERO-Zellkulturen und der AVIVER-Zellen wird vorzugsweise ein vollsynthetisches Medium verwendet, besonders bevorzugt ist das Medium MEM ("minimal essential medium") plus 5 bis 20%, vorzugsweise 10% BMS (Serumersatzmedium) und 5 bis 20, vorzugsweise 10% Lactalbuminhydrolysat. Als Virusmedium wird nach dem Austausch mit dem Anzuchtmedium, vorzugsweise MEM-Medium, mit 5 bis 20%, vorzugsweise mit 10% Lactalalbuminhydrolysat, ohne BMS bzw. ohne fetalem Kälberserum und ohne Antibiotika verwendet. Sämtliche Herstellungsverfahren werden vorzugsweise bei pH-Werten von 7.0 bis 8.0, vorzugsweise bei einem pH-Wert von 7,25 durchgeführt. Virusernten mit Titer von 10^{5.0} bis 10^{7.5}, vorzugsweise von mindestens 10^{6.5} TCID₅₀/ml (TCID₅₀ = 50% tissue culture infectious dose), sind bevorzugt als Ausgangsmaterial für die Herstellung des erfindungsgemäßen Monoparamunitätsinducers PIND-MYXO geeignet.

Die Vermehrung des Myxomavirus führt in VERO-Zellen zu einem typischen cytopathischen Effekt (cpE), der schließlich durch eine Zerstörung der infizierten Zellen (Lyse) charakterisiert ist. Bei einer Animpfdosis von ca. 10 MOI ("multiplicity of infection") kommt es nach einer kurzen Abkugelungsphase (1-2 Tage) für etwa 3 Tage zu netzartigen Zellstrukturen und nach etwa 5 Tagen zur Lyse der Zellen. Die 301. Passage in VERO-Zellen hatte einen Infektionstiter von etwa 10^{6.5} TCID₅₀/ml.

Für die Inaktivierung des attenuiertem Myxomavirus wird eine chemische Behandlung mit Beta-Propiolacton bei einer Konzentration von 0,01-1% Beta-Propiolacton, vorzugsweise bei einer Konzentration von 0,05% Beta-Propiolacton durchgeführt. Diese Inaktivierung führt zu einem vollständigen Verlust der ggf. noch vorhandenen immunisierenden Eigenschaften, wobei die paraspezifischen Aktivitäten nicht nur erhalten bleiben, sondern sogar signifikant ansteigen.

Zur weiteren Verarbeitung der attenuierten und inaktivierten Myxomaviren zu einem Myxomavirus-Monoparamunitätsinducer (PIND-MYXO) sollte das zur Virusinaktivierung benutzte Virusausgangsmaterial einen Virustiter von etwa 10^{5.0} bis 10^{7.0}, vorzugsweise von mindestens 10^{6.5} TCID₅₀/ml, haben.

Die Reinigung erfolgt vorzugsweise mittels Zentrifugation bei niedriger Umdrehungszahl (z.B. 1000 UpM). Nach der Zentrifugation wird 0,5-10% succinylierte Gelatine (z.B. Polygeline, z.B. Firma Hausmann, St. Gallen/Schweiz), vorzugsweise 5% succinylierte Gelatine zugesetzt. Das resultierende Gemisch kann anschließend in Portionen von 1.5 ml in entsprechenden sterilen Glasfläschchen oder Ampullen lyophilisiert und bei Bedarf mit destilliertem Wasser (Aqua dest.) aufgelöst werden. Ein Volumen von 0,5-2 ml, vorzugsweise von 1,0 ml des mit Aqua dest. aufgelösten Lyophilisats entspricht einer Impfdosis für den Menschen bei intramuskulärer Applikation (siehe auch Mayr A. und Mayr. B.: "Von der Empirie zur Wissenschaft", Tierärztl. Umschau, Aufl. 57: 583-587, 2002).

Klinisch kann die Attenuierung durch den Verlust der Virulenz für europäische Haus- und Wildkaninchen (Gattung *Oryctolagus caniculus csp.*), durch einen Verlust der Kontagiosität, durch eine nahezu vollständige Einengung des Wirtsspektrums in Zellkulturen und durch die Änderung der immunisierenden Eigenschaften nachgewiesen werden.

Das lyophilisierte Präparat kann bei Temperaturen von vorzugsweise etwa +4°C oder bei geringeren Temperaturen, vorzugsweise etwa -60°C, zeitlich unbegrenzt stabil gelagert werden.

Durch gentechnologische Untersuchungen der präparierten attenuierten Myxomaviren wurde nachgewiesen, dass es im Myxomavirus-Genom zu multiplen Deletionen gekommen war. Im Falle des Ausgangsstammes M-2 besteht das Myxomavirusgenom aus einer einzelnen linearen Desoxyribonukleinsäure (DNA) mit einer Länge von insgesamt etwa 160 Kilobasen (kB), die für mehrere hundert Proteine kodiert (Herrlich A., Mayr A. und Munz E.: "Die Pocken", 2. Aufl., Georg Thieme Verlag, Stuttgart, 1967). Die Sequenzen der terminal gelegenen "Inverted Repeats" (engl.: "terminal inverted repeats", TIR) liegen bei etwa 11 kB des Genomabschnitts (McFadden, G. and Graham, K.: "Modulation of cytokine networks by poxvirus", Virology, Aufl. 5: 421-429, 1994).

So wurde festgestellt, dass es durch die Attenuierung der Myxomaviren über kontinuierliche Vero-Zell-Passagen zu einem Verlust der kodierenden Genabschnitte für die Rezeptoren für Interferon α und γ (IFN α, IFNγ), für den Tumornekrosefaktor (TNF) und für die Interleukine (IL-) 1, 2, 6 und 12 gekommen ist. Interessanterweise zählen diese Zytokine zu den paraspezifischen Abwehrfaktoren des unspezifischen Immunsystems. Durch Bindung an die entsprechenden Virus-Rezeptoren werden die Zytokine neutralisiert, so dass sich das Virus ungehemmt vermehren kann. Die Deletionen von Genabschnitten, die für die oben genannten Zytokin-Rezeptoren kodieren, betreffen hauptsächlich die terminalen Bereiche der DNA. Daneben konnte man aber auch im konservierten Teil der DNA Deletionen nachweisen, die während den AVIVER-Zellpassagen entstanden sind. Diese Deletionen betreffen zwei Gene, die für ein Immunepitop und Virulenzgen kodieren. Vermutlich sind solche genetische Modifikationen mit ein Grund für die Abnahme der immunisierenden, d.h. antigenspezifischen Aktivitäten und die gleichzeitige Zunahme der paraspezifischen Aktivitäten des attenuierten Myxomavirus.

Die immunisierenden Epitope und die paraspezifischen bzw. unspezifischen Epitope sind konkurrierend. Deshalb führt eine Abnahme der erstgenannten Peptide oder Proteine zu einem Anstieg der Wirkung der paraspezifischen Aktivitäten. Reste immunisierender und virulenzsteigernder Proteine werden bei der Präparation von Monoparamunitätsinducern durch das oben beschriebene Verfahren zur Inaktivierung der attenuierten Myxomaviren beseitigt.

Der erfindungsgemäße Monoparamunitätsinducer, auch PIND-MYXO genannt, basiert auf der Verwendung von attenuierten Myxomaviren oder deren paramunisierenden Bestandteilen und ist aufgrund seiner paramunisierenden Eigenschaften für folgende prophylaktische oder therapeutische Indikationen bei einem Patienten geeignet:
- Infektiöse Faktorenkrankheiten und Mischinfektionen, chronische Manifestationen infektiöser Prozesse, hartnäckig rezidivierende Infektionen und chemotherapieresistente, bakterielle und virale Infektionen
- Abwehrschwächen beziehungsweise Dysregulationen im Abwehrsystem eines Organismus
- neonatale Infektionsbedrohung
- adjuvante Therapie bei bestimmten Tumorkrankheiten, z.B. Verhütung der Metastasierung, Minderung von Nebenwirkungen durch Chemo- und Strahlentherapie
- Regulierung der Homöostase zwischen Hormon-, Kreislauf-, Stoffwechsel- und Nervensystem.

Die erfindungsgemäßen Paramunitätsinducer können Säugetieren, einschließlich dem Menschen, Vögeln und Reptilien parenteral oder lokal verabreicht werden. Die lokale Verabreichung von Paramunitätsinducern stimuliert speziell die paraspezifischen Abwehrmechanismen in den Schleimhäuten und in der Haut. Daneben kommt es aber auch zu einer gewissen systemischen Wirkung. Dagegen beeinflussen parenteral angewendete Paramunisierungen die lokalen Abwehrmechanismen in der Haut und der Schleimhaut kaum, wobei sie bevorzugt systemisch wirken.

Nebenwirkungen traten selbst bei zahlreichen, kontinuierlich durchgeführten, parenteralen Applikationen bei Mensch und Tier nicht auf. Für den Einsatz von PIND-MYXO gelten die gleichen Indikationen beim Tier wie beim Menschen. Zugleich empfiehlt es sich, in Problembetrieben, speziell bei Haltungen von Pferden, Schweinen, Hunden und Katzen, die Neugeborenen sofort am Tage der Geburt und vorzugsweise am 1. und eventuell auch 2. Tag nach der Geburt zu paramunisieren. Die Einzeldosis beträgt etwa 0,5 bis 5 ml des aufgelösten Lyphilisats, bei Pferd und Schwein beträgt die Einzeldosis vorzugsweise 2 ml und bei Hund und Katze vorzugsweise 0,5 ml bei parenteraler Applikation. Erfindungsgemäß empfiehlt es sich, zur Vermeidung von Nebenreaktionen und zur Unterstützung der Immunisierung bei der Verabreichung von Impfstoffen PIND-MYXO einen Tag vor und/oder gleichzeitig mit spezifischen Schutzimpfungen parenteral zu verabreichen.

Eine Ausführungsform der Erfindung betrifft die Herstellung einer pharmazeutischen Zusammensetzung zur lokalen Applikation für die Induktion der Paramunität in Haut und Schleimhäuten. Vorzugsweise betrifft die pharmazeutische Zusammensetzung eine Buccal- oder Lutschtablette basierend auf Bestandteilen eines attenuierten und inaktivierten Myxoma-Zellkulturvirus. Die erfindungsgemäßen Buccal-Tabletten werden bevorzugt unter Zusatz von Sorbit, Polyethylenglykol 6,00, Kaliumhydrogenphosphat, Tyrospirol-Tablettenessenz, Kollidon 25 und Magnesiumstearat hergestellt. PIND-MYXO kann aber auch nasal, rektal oder vaginal mit geeigneten Trägern verabreicht werden.

Die nachfolgenden Beispiele sind bevorzugte Ausführungsformen der Erfindung und dienen der weiteren Erläuterung des Erfindungsgegenstandes.

### Beispiel 1

Als Ausgangsmaterial zur Herstellung des erfindungsgemäßen Monoparamunitätsinducers PIND-MYXO wurde aus der ödematösen Subkutis eines in typischer Weise an Myxomatose erkrankten europäischen Wildkaninchens (Gattung *Oryctolagus*) das Myxomavirus über die Anzüchtung auf der Chorioallantoismembran (CAM) 10 Tage bebrüteter Hühnereier (VALO-Eier) isoliert und dreimal nach dem Verfahren von Herrlich *et al.* auf der CAM in Passagen adaptiert (Herrlich A., Mayr A. und Munz E.: "Die Pocken", 2. Aufl., Georg Thieme Verlag, Stuttgart, 1967). Die dritte CAM-Passage wurde in einer 1. Stufe auf VERO-Zellen über 120 Passagen (ATCC CCL-81, WHO, American Type Culture Collection) adaptiert, in einer 2. Stufe durch 24 Zwischenpassagen in AVIVER-Zellkulturen vermehrt und in der 3. Phase in VERO-Zellen weitergezüchtet. Insgesamt sind etwa 300 Passagen mit dem Ziel der Attenuierung durchgeführt worden. Nach diesen kontinuierlichen Endverdünnungspassagen war das ursprünglich virulente Myxomavirus attenuiert.

Das attenuierte Myxomavirus wird in Vero-Zellen vermehrt. Für die Anzucht der Vero-Zellkulturen wird ein vollsynthetisches Medium, bestehend aus MEM ("minimal essential medium") plus 10% BMS (Serumersatzmedium) und 10% Lactalbuminhydrolysat verwendet. Als Virusmedium wird nach dem Austausch mit dem Anzuchtmedium nur MEM mit 10% Lactalbuminhydrolysat ohne BMS bzw. ohne fetales Kälberserum und ohne Antibiotika verwendet. Sämtliche Herstellungsverfahren werden bei pH-Werten von über 7,25 durchgeführt. Virusernten mit Titer über 10^{6.5} TCID₅₀/ml dienen als Ausgangsmaterial für die Herstellung des erfindungsgemäßen Monoparamunitätsinducers PIND-MYXO. Nach Inaktivierung der Virusernten mit 0,05% Beta-Propiolacton und grobtouriger Zentrifugation, wird dem Virusmaterial 5% succinylierte Gelatine (Polygeline) vor der Lyophilisierung zugesetzt.

Das lyophilisierte Präparat hält sich bei Zimmertemperaturen wie auch bei Temperaturen von etwa 4°C bis -80° C und ist vorzugsweise bei etwa 4°C oder auch etwa -60°C zeitlich unbeschränkt haltbar. Ein Volumen von 1 ml des in sterilem Aqua dest. aufgelösten Lyophilisats entspricht einer Impfdosis. Die Applikation erfolgt tief intramuskulär bzw. lokal (siehe Beispiele 3, 4 und 5).

### Beispiel 2

In analoger Weise wie in Beispiel 1 beschrieben, wird der erfindungsgemäße PIND-MYXO-Inducer in trockener Form (Lyophilisat nicht aufgelöst) lokal auf die Schleimhäute des oberen Respirationstrakts, bevorzugt nasal, dreimal am Tag zur Prophylaxe bzw. Therapie (je 1 ml pro Applikation) von multifaktoriellen Infektionen (z.B. grippale Infekte) appliziert.

### Beispiel 3

In analoger Weise wird der wie in Beispiel 1 hergestellte PIND-MYXO-Inducer in flüssiger Form erfindungsgemäß kutan zur besseren Durchblutung der Haut, zur schnelleren Abheilung von Wunden sowie zur Behandlung von Krampfadern bzw. chronisch-venösen Insuffizienzen (Ulcus cruris) beim Menschen eingerieben. Das Lyophilisat kann zu diesem Zweck zum Beispiel in Fettcreme (z.B. Bepanthen, Linola-Fett) aufgenommen werden, wobei der pH-Wert leicht alkalisch sein sollte. Diese Zubereitung sollte für jede Anwendung frisch hergestellt werden. Die Applikation wird mehrmals am Tage durch manuelles Einreiben der unverletzten Haut vorgenommen. Offene Wunden können durch Aufträufeln des frisch gelösten Präparates auf die Wundbereiche behandelt werden. Die Behandlung sollte täglich bis zur Heilung erfolgen.

### Beispiel 4

Analog wird der wie in Beispiel 1 hergestellte Monoparamunitätsinducer PIND-MYXO zur Vermeidung von Nebenreaktionen und zur Verbesserung des Impferfolges einen Tag vor und gleichzeitig bei einer Schutzimpfung mit klassischen, spezifischen Vaccinen parenteral verabreicht.

### Beispiel 5

Analog wird der wie in Beispiel 1 hergestellte Monoparamunitätsinducer PIND-MYXO zu Buccal- bzw. Lutschtabletten verarbeitet. Die Herstellung und Verwendung der Lutschtabletten für die lokale Paramunisierung der Hals-Nasen-Ohren- und Mundschleimhäute ist neu und Bestandteil der Erfindung. Über die aktivierten Mundschleimhäute kommt es nicht nur zu einem "homing"-Effekt (Auswandern von Abwehrzellen in Schleimhäute anderer Organsysteme), sondern auch zu einer partiellen parenteralen Paramunisierung. Für die Herstellung der Buccal- bzw. Lutschtabletten hat sich folgendes Herstellungsverfahren bewährt:

Dem flüssigen Inducermaterial wird zur Lyophilisierung an Stelle von Gelatine, 5% Kollidon 25 (Polyvinylpyrrolidon) zugesetzt. Zur Herstellung der fertigen Tablette benötigt man Harnstoff, Sorbit, Polyethylenglykol 6000 und Magnesiumstearat. Als Rezeptur für eine Tablette mit einem Gewicht von 500,5 mg Gewicht wird empfohlen:

| | |
|---|---|
| PIND-MYXO-Lyophilisat | 65 mg |
| Harnstoff | 50 mg |
| Sorbit | 267 mg |
| Polyethylenglykol 6000 | 118 mg |
| Magnesiumstearat | 0,5 mg |
| Tablettengewicht | 500,5 mg |

Pro Tag sollten zur Erzielung einer optimalen Paramunisierung 4-6 Tabletten von dem Patienten in regelmäßigen Abständen eingenommen werden.

Folgende Rezeptur einer pharmazeutischen Zusammensetzung hat sich für die Herstellung von Buccal-Tabletten bewährt:

| | |
|---|---|
| PIND-MYXO-Lyophilisat | 115 mg |
| Sorbit | 360 mg |
| Polyethylenglykol 6000 | 300 mg |
| Kaliumdihydrogenphosphat (KH₂PO₄), wasserfrei | 2 mg |
| Dinatriumhydrogenphosphat (Na₂HPO₄), wasserfrei | 8 mg |
| Tyrospirol-Essenz-Tabletten | 0,8 mg |
| Magnesiumstearat | 20 mg |
| Tablettengewicht | 805,8 mg |

Die Tabletten lösen sich im Mund des Patienten langsam auf und können nach der Auflösung geschluckt werden.

Die in den Tabellen 1 bis 4 zusammengestellten klinischen Versuchsergebnisse mit dem erfindungsgemäßen Monoparamunitätsinducer, basierend auf dem Lyophilisat des Myxomavirus-Stammes M-2, demonstrieren die sehr guten paramunisierenden Aktivitäten von Myxomaviruslyophilisaten beim Menschen. Diese Daten sind gleichermaßen auf andere Säugetiere wie auch auf Vögel und Reptilien übertragbar.

**Tabelle 1:**

| **Klinische Ergebnisse mit einem Monoparamunitätsinducer aus attenuiertem** **Myxoma-Zellkulturvirus beim Menschen** **- prophylaktische Anwendungen -** **(lyphilisierter Inducer 1 OP (1 ml) intramuskulär)** | |
|---|---|
| **Indikationen** | **bewährte Applikationsmethoden** |
| Zeiten mit starkem Infektionsdruck | |
| Stress | 2 Injektionen vor der Belastung |
| Reisen, Prüfungen und ähnliche Belastungen | im Abstand von 24 Stunden |
| vor bzw. gleichzeitig mit Schutzimpfungen | |
| Chemotherapie, Bestrahlung | |
| (Minderung bzw. Verhinderung von Nebenreaktionen) | 1 Injektion täglich oder jeden 2. Tag bis zum |
| Operationen | Abschluss der Behandlung bzw. bis zur |
| (Verbesserung der Wundheilung) | Genesung |
| Aufrechterhaltung einer optimalen Abwehr | |
| bzw. Homöodynamik | pro Monat 1-2 Injektionen im Abstand von 24 Stunden |
| Vorsorge gegen Krebs und Hepatitiden | |
| Verbesserung des Wohlbefindens | |

**Tabelle 2:**

| **Klinische Ergebnisse mit einem Monoparamunitätsinducer aus attenuiertem Myxoma-** **Zellkulturvirus beim Menschen** **- therapeutische Anwendung -** **(lyphilisierter Inducer 1 OP (1 ml) intramuskulär)** | |
|---|---|
| **Indikationen** | **bewährte Applikationsmethoden** |
| Herpeskrankheiten | täglich 1 Injektion über 3-5 Tage bzw. bis zum Abklingen der Symptome; anschließend |
| (Zoster, infektiöse Mononukleose, Herpes simplex, usw.) | jeden 2. bzw. 3. Tag 1 Injektion bis zur völligen Genesung |
| chronische Hepatitiden | pro Monat eine "Kur": 3 Injektionen im |
| | Abstand von jeweils 24 Stunden |
| grippale Infekte virale sowie bakterielle Mischinfektionen | 1 Applikation pro Tag bis zum Abklingen der Symptome, anschließend 1 Injektion jeden 2. |
| (in Kombination mit Antibiotika- bzw. Chemotherapie) | Tag bis zur völligen Genesung |
| Immunschwächen und Dysregulation der | 1. Intensivbehandlung über 5-10 Tage: |
| Abwehrsysteme (z.B. während bzw. nach | 1 Injektion pro Tag |
| Chemotherapie) | 2. anschließend 2 Injektionen pro Woche im Abstand von 24 Stunden |
| | (Behandlung über längeren Zeitraum möglich) |
| Endotoxinschäden | täglich 1 Injektion über 7 Tage bzw. bis zur Genesung |

**Tabelle 3:**

| **Wirkung einer Paramunisierung mit Myxoma-Inducer (PIND-MYXO) bei Patienten mit niedrigen Immunparametern (7 Tage nach PIND-Myxo-Applikation)** | | | | |
|---|---|---|---|---|
| **Patient** | **Patienten-Daten** **Diagnose/Therapie** | **Parameter** **(Normbereich)** | **Tag 0** | **Tag 7** |
| A.S. | | | | |
| weibl., 52 Jahre | Immunsuppression | Leukozyten | 4000 | 9400 |
| | | (4000-10000/µl) | | |
| D.B. | Colitis ulcerosa | Lymphozyten | 620 | 1360 |
| weibl., 54 Jahre | Cortisontherapie | (900-3000/µl) | | |
| | | CD4-Zellen | 400 | 920 |
| | | (500-1800/µl) | | |
| | | CD8-Zellen | 80 | 210 |
| | | (100-1000/µl) | | |
| U.S. | Immunstörung | Leukozyten | 3800 | 7400 |
| männl., 38 Jahre | | (4000-10000/µl) | | |
| S.C. | mestastasierendes | Leukozyten | 3800 | 9900 |
| männl., 43 Jahre | Prostata-CA Radiotherapie | (4000-10000/µl) | | |
| B.M. | | | | |
| weibl., 56 Jahre | Infektanfälligkeit | zytotoxische Zellen | 0 | 248 |
| | | (30-360/µl) | | |

**Tabelle 4:**

| **Wirkung einer Paramunisierung mit Myxo-Inducer (PIND-MYXO) bei Patienten** **mit erhöhten Immunparametern (7 Tage nach PIND-MYXO-Applikation)** | | | | |
|---|---|---|---|---|
| **Patient** | **Diagnose/Therapie** | **Parameter** **(Normbereich)** | **Tag 0** | **Tag 7** |
| G.P., | Cervix-, Mamma- | Leukozyten | | |
| weibl., 59 Jahre | CA, | (4000-10000/µl) | 12600 | 8600 |
| | Infektanfälligkeit | Granulozyten | | |
| | | (2400-6400/µl) | 8420 | 5930 |
| C.H. | Psychosomatisches | Leukozyten | 12700 | 6000 |
| weibl., 51 Jahre | Syndrom, | (4000-10000/µl) | | |
| | Adipositas | Granulozyten | 9270 | 4760 |
| | | (2400-6400/µl) | | |

Die Erfindung ist weiterhin durch die folgenden Gegenstände charakterisiert
1. Monoparamunitätsinducer auf der Basis von paramunisierenden Viren oder Viruskomponenten, **dadurch gekennzeichnet, dass** die Viren oder Viruskomponenten von einem attenuierten Myxomavirus-Stamm des Kaninchens stammen.
2. Monoparamunitätsinducer nach Gegenstand 1, **dadurch gekennzeichnet, dass** der attenuierte Myxomavirus-Stamm eine Modifikation in Form einer Addition, Substitution oder Deletion in einem oder mehreren Genabschnitten, kodierend für die Bildung von Zytokin-Rezeptoren, aufweist, wobei durch die Modifikation die Rezeptoreigenschaften des Zytokin-Rezeptors verloren geht.
3. Monoparamunitätsinducer nach Gegenstand 2, **dadurch gekennzeichnet, dass** die Zytokin-Rezeptoren ausgewählt sind aus der Gruppe von Rezeptoren für Interferone (IFN), Interleukine (IL) und Tumornekrosefaktor (TNF).
4. Monoparamunitätsinducer nach Gegenstand 3, **dadurch gekennzeichnet, dass** die Zytokin-Rezeptoren ausgewählt sind aus der Gruppe von Rezeptoren für IFNα-R, IFNγ-R, TNF-R, IL-1-R, IL-2-R, IL-6-R, IL-12-R.
5. Monoparamunitätsinducer nach einem der Gegenstände 1 bis 4, **dadurch gekennzeichnet, dass** die Viruskomponenten Virushüllen oder aberrante Formen von Virushüllen eines attenuierten Myxomavirus-Stammes umfassen.
6. Monoparamunitätsinducer nach einem der Gegenstände 1 bis 5, **dadurch gekennzeichnet, dass** der Myxomavirus-Stamm ausgewählt ist aus der Gruppe bestehend aus M2, M7, Lausanne, Aust/Uriarra/Verg-86/1.
7. Monoparamunitätsinducer nach Gegenstand 6, **dadurch gekennzeichnet, dass** es sich bei dem Myxomavirus-Stamm um den Stamm M-2 mit der Hinterlegungsnummer ECACC 03121801 handelt.
8. Monoparamunitätsinducer nach einem der Gegenstände 1 bis 7, **dadurch gekennzeichnet, dass** der Myxomavirus-Stamm in Zellkulturen attenuiert wurde.
9. Monoparamunitätsinducer nach Gegenstand 8, **dadurch gekennzeichnet, dass** es sich bei den Zellkulturen um Vero-Affennierenzellen und/oder AVIVER-Zellen handelt.
10. Monoparamunitätsinducer nach einem der Gegenstände 1 bis 9, **dadurch gekennzeichnet, dass** die attenuierten Myxomaviren inaktiviert wurden.
11. Monoparamunitätsinducer nach Gegenstand 10, **dadurch gekennzeichnet, dass** die attenuierten Myxomaviren mit Beta-Propiolacton inaktiviert wurden.
12. Monoparamunitätsinducer nach Gegenstand 11, **dadurch gekennzeichnet, dass** die Konzentration von Beta-Propiolacton 0,01-1 % beträgt.
13. Monoparamunitätsinducer nach Gegenstand 12, **dadurch gekennzeichnet, dass** die Konzentration von Beta-Propiolacton 0,05% beträgt.
14. Monoparamunitätsinducer nach einem der Gegenstände 1 bis 13, **dadurch gekennzeichnet, dass** die Myxomaviren lyophilisiert wurden.
15. Verwendung des Monoparamunitätsinducers nach einem der Gegenstände 1 bis 14 für die Herstellung einer pharmazeutischen Zusammensetzung.
16. Verwendung des Monoparamunitätsinducers nach einem der Gegenstände 1 bis 14 für die Herstellung einer pharmazeutischen Zusammensetzung zur Aktivierung des paraspezifischen Immunsystems bei Mensch und Tier.
17. Verwendung des Monoparamunitätsinducers nach einem der Gegenstände 1 bis 14 für die Herstellung einer pharmazeutischen Zusammensetzung zur parenteralen Behandlung und/oder zur Prophylaxe von Dysfunktionen des Immunsystem, Immunsuppression, Immunschwäche-Erkrankungen, Dysfunktionen der Homöostase zwischen Hormon-, Kreislauf-, Stoffwechsel- und Nervensystem, neonataler Infektionsbedrohung, Tumorerkrankungen, Viruserkrankungen, bakteriellen Erkrankungen, therapieresistenten infektiösen Faktorenkrankheiten, viralen und bakteriellen Mischinfektionen, chronischen Manifestationen infektiöser Prozesse, Leberkrankheiten unterschiedlicher Genese, chronischen Hautkrankheiten, Herpeskrankheiten, chronischen Hepatitiden, grippalen Infekten, Endotoxinschäden.
18. Verwendung nach einem der Gegenstände 15 bis 17, wobei die Behandlung durch eine lokale Verabreichung der pharmazeutischen Zusammensetzung über die Haut oder Schleimhäute eines Patienten erfolgt.
19. Pharmazeutische Zusammensetzung, umfassend einen Monoparamunitätsinducer nach einem der Gegenstände 1 bis 14 und einen pharmazeutisch verträglichen Träger.
20. Pharmazeutische Zusammensetzung nach Gegenstand 19, zur lokalen oder parenteralen Applikation.
21. Pharmazeutische Zusammensetzung nach Gegenstand 19, wobei die Zusammensetzung in Form von Buccal- und Lutschtabletten vorliegt.
22. Verfahren zur Herstellung eines Monoparamunitätsinducers basierend auf einem attenuierten Myxomavirus-Stamm des Kaninchens, umfassend die Schritte:
   - Isolieren von Myxomaviren aus infiziertem Gewebe eines typisch an einer generalisierten Myxomatose erkrankten Kaninchens;
   - Adaptierung des Virus an ein permissives Zellsystem;
   - Passagierung der isolierten Viren in einer permissiven Zellkultur, bis eine Attenuierung des Virus erreicht ist.
23. Verfahren nach Gegenstand 22, wobei zur Adaptierung die isolierten Viren auf die Chorioallantoismembran (CAM) bebrüteter Hühnereier geimpft werden.
24. Verfahren nach Gegenstand 23, wobei das Virus über 2 bis 6 Passagen, vorzugsweise über 3 Passagen, auf der CAM vermehrt wird.
25. Verfahren nach Gegenstand 22, wobei zur Isolierung der im infizierten Gewebe enthaltenden Myxomaviren diese in einem permissiven Zellsystem vermehrt werden.
26. Verfahren nach Gegenstand 25, **dadurch gekennzeichnet, daß** das Virus durch Anzucht auf der Chorioallantoismembran (CAM) bebrüteter Hühnereier isoliert wird.
27. Verfahren nach Gegenstand 26, **dadurch gekennzeichnet, daß** das Virus anschließend an die CAM über weitere Passagen, vorzugsweise über 2 weitere Passagen, adaptiert wird.
28. Verfahren nach einem der Gegenstände 22 bis 27, wobei das Virus in einer permanenten Zellkultur passagiert wird.
29. Verfahren nach Gegenstand 28, wobei das Virus in einer Vero-Zellkultur passagiert wird.
30. Verfahren nach Gegenstand 29, **dadurch gekennzeichnet, dass** die Attenuierung der Myxomaviren über 80 bis 150 Passagen, vorzugsweise über 120 Passagen, in Vero-Zellkulturen erfolgt.
31. Verfahren nach einem der Gegenstände 28 bis 30, wobei das Virus in einer binären Zellkultur passagiert wird.
32. Verfahren nach Gegenstand 31, wobei das Virus in einer AVIVER-Zellkultur passagiert wird.
33. Verfahren nach Gegenstand 32, **dadurch gekennzeichnet, dass** die Passagierung über 10 bis 50 Passagen, vorzugsweise über 25 Passagen, erfolgt.
34. Verfahren nach einem der Gegenstände 22 bis 33, wobei die attenuierten Myxomaviren zusätzlich über weitere Attenuierungspassagen vermehrt werden.
35. Verfahren nach Gegenstand 34, wobei die Vermehrung in Vero-Affennierenzellen erfolgt.
36. Verfahren nach Gegenstand 35, **dadurch gekennzeichnet, dass** die Weitermehrung in Vero-Zellen über 100 bis 200 Passagen erfolgt.
37. Verfahren nach einem der Gegenstände 34 bis 36, **dadurch gekennzeichnet, dass** die Virusernten der Zellkultur einen Infektionstiter von 10⁵ bis 10^{7.5}, vorzugsweise von mindestens 10^{6.5} , TCID₅₀/ml haben.
38. Verfahren nach einem der Gegenstände 22 bis 37, wobei die attenuierten Myxomaviren zusätzlich inaktiviert werden.
39. Verfahren nach Gegenstand 38, wobei die attenuierten Myxomaviren zur Inaktivierung mit Beta-Propiolacton behandelt werden.
40. Verfahren nach Gegenstand 39, **dadurch gekennzeichnet, dass** die Konzentration von Beta-Propiolacton 0,01-1% beträgt.
41. Verfahren nach Gegenstand 40, **dadurch gekennzeichnet, dass** die Konzentration von Beta-Propiolacton 0,05% beträgt.
42. Verfahren nach einem der Gegenstände 22 bis 41, umfassend die Schritte:
   - Isolieren von Myxomaviren aus dem infizierten Gewebe des an Myxomatose erkrankten Kaninchens durch Vermehrung auf der CAM bebrüteter Hühnereier und nachfolgender
   - Adaptierung des isolierten Myxomavirus an die CAM über weitere 2 Passagen
   - Attenuieren der isolierten Viren durch
      - Passagierung in Vero-Zellkulturen, vorzugsweise über 120 Passagen,
      - Überführung der Viren in die binäre AVIVER-Zellkultur mit weiterer Attenuierung des Virus, vorzugsweise über 24 Zwischenpassagen, in dieser Zellkultur,
      - nachfolgende Rückübertragung des Virus auf Vero-Affennierenzellen, und
   - Vermehren der attenuierten Myxomaviren durch weitere Attenuierungspassagen in den Vero-Zellen, vorzugsweise über etwa 150 Passagen.
43. Attenuiertes Myxomavirus, erhältlich durch das Verfahren nach einem der Gegenstände 22 bis 42.
44. Myxomavirus nach Gegenstand 43, hinterlegt bei der European Collection of Cell Cultures (ECACC) unter der Hinterlegungsnummer 03121801.
45. Myxomavirus nach Gegenstand 43 oder 44, **dadurch gekennzeichnet, dass** das Myxomavirus genetisch modifiziert ist.
46. Myxomavirus nach Gegenstand 45, **dadurch gekennzeichnet, dass** das Myxomavirus eine Modifikation in Form einer Addition, Substitution oder Deletion in einem oder mehreren Genabschnitten, kodierend für die Bildung von Zytokin-Rezeptoren aufweisen, wobei durch die Modifikation die Rezeptoreigenschaften der Zytokin-Rezeptoren verloren gehen.
47. Myxomavirus nach Gegenstand 46, **dadurch gekennzeichnet, dass** die Zytokin-Rezeptoren ausgewählt sind aus der Gruppe von Rezeptoren für Interferone (IFN), Interleukine (IL) und Tumornekrosefaktor (TNF).
48. Myxomavirus nach Gegenstand 47, **dadurch gekennzeichnet, dass** die Zytokin-Rezeptoren ausgewählt sind aus der Gruppe von Rezeptoren für IFNα-R, IFNγ-R, TNF-R, IL-1-R, IL-2-R, IL-6-R, IL-12-R.

## Patentansprüche

1. Attenuiertes Myxomavirus oder Komponente davon zur Verwendung als paramunisierender Wirkstoff.

2. Verwendung eines attenuierten Myxomavirus oder Komponente davon für die Herstellung einer pharmazeutischen Zusammensetzung enthaltend das attenuierte Myxomavirus oder Komponente davon als paramunisierenden Wirkstoff.

3. Pharmazeutische Zusammensetzung, umfassend ein attenuiertes Myxomavirus oder Komponente davon als paramunisierenden Wirkstoff und einen pharmazeutisch verträglichen Träger.

4. Attenuiertes Myxomavirus oder Komponente davon nach Anspruch 1, Verwendung nach Anspruch 2 und/oder pharmazeutische Zusammensetzung nach Anspruch 3, wobei das Myxomavirus genetisch modifiziert ist.

5. Attenuiertes Myxomavirus oder Komponente davon, Verwendung und/oder pharmazeutische Zusammensetzung nach Anspruch 4, wobei das attenuierte Myxomavirus eine Modifikation in Form einer Addition, Substitution oder Deletion in einem oder mehreren Genabschnitten, kodierend für die Bildung von Zytokin-Rezeptoren, aufweist, wobei durch die Modifikation die Rezeptoreigenschaften des Zytokin-Rezeptors verloren gehen.

6. Attenuiertes Myxomavirus oder Komponente davon, Verwendung und/oder pharmazeutische Zusammensetzung nach Anspruch 5, wobei die Zytokin-Rezeptoren ausgewählt sind aus der Gruppe von Rezeptoren für Interferone (IFN), Interleukine (IL) und Tumornekrosefaktor (TNF).

7. Attenuiertes Myxomavirus oder Komponente davon, Verwendung und/oder pharmazeutische Zusammensetzung nach einem der Ansprüche 5 oder 6, wobei die Zytokin-Rezeptoren ausgewählt sind aus der Gruppe von Rezeptoren für IFNα-R, IFNγ-R, TNF-R, IL-1-R, IL-2-R, IL-6-R, IL-12-R.

8. Attenuiertes Myxomavirus oder Komponente davon, Verwendung und/oder pharmazeutische Zusammensetzung nach einem der Ansprüche 1 bis 7, wobei die Viruskomponenten Virushüllen oder aberrante Formen von Virushüllen eines attenuierten Myxomavirus-Stammes umfassen.

9. Attenuiertes Myxomavirus oder Komponente davon, Verwendung und/oder pharmazeutische Zusammensetzung nach einem der Ansprüche 1 bis 8, wobei der Myxomavirus-Stamm ausgewählt ist aus der Gruppe bestehend aus M2, M7, Lausanne, Aust/Uriarra/Verg-86/1.

10. Attenuiertes Myxomavirus oder Komponente davon, Verwendung und/oder pharmazeutische Zusammensetzung nach einem der Ansprüche 1 bis 9, wobei der Myxomavirus-Stamm bei der European Collection of Cell Cultures (ECACC) unter der Hinterlegungsnummer 03121801 hinterlegt ist.

11. Attenuiertes Myxomavirus oder Komponente davon, Verwendung und/oder pharmazeutische Zusammensetzung nach einem der Ansprüche 1 bis 10, wobei das Myxomavirus in Zellkulturen, vorzugsweise in Zellkulturen von Vero-Affennierenzellen und/oder AVIVER-Zellen, attenuiert wurde.

12. Attenuiertes Myxomavirus oder Komponente davon, Verwendung und/oder pharmazeutische Zusammensetzung nach einem der Ansprüche 1 bis 11, wobei die attenuierten Myxomaviren inaktiviert wurden, vorzugsweise mit Beta-Propiolacton.

13. Attenuiertes Myxomavirus oder Komponente davon, Verwendung und/oder pharmazeutische Zusammensetzung nach einem der Ansprüche 1 bis 12 zur Aktivierung des paraspezifischen Immunsystems bei Mensch und Tier.

14. Attenuiertes Myxomavirus oder Komponente davon, Verwendung und/oder pharmazeutische Zusammensetzung nach einem der Ansprüche 1 bis 13 zur parenteralen Behandlung und/oder zur Prophylaxe von Dysfunktionen des Immunsystem, Immunsuppression, Immunschwäche-Erkrankungen, Dysfunktionen der Homöostase zwischen Hormon-, Kreislauf-, Stoffwechsel- und Nervensystem, neonataler Infektionsbedrohung, Tumorerkrankungen, Viruserkrankungen, bakteriellen Erkrankungen, therapieresistenten infektiösen Faktorenkrankheiten, viralen und bakteriellen Mischinfektionen, chronischen Manifestationen infektiöser Prozesse, Leberkrankheiten unterschiedlicher Genese, chronischen Hautkrankheiten, Herpeskrankheiten, chronischen Hepatitiden, grippalen Infekten, Endotoxinschäden.
